# EUROPEAN PATENT SPECIFICATION

(11) **EP 1 647 278 B1**
(45) Date of publication and mention of the grant of the patent: **26.08.2009**
(21) Application number: 05254444.2
(22) Date of filing: 15.07.2005
(51) Int. Cl.: A61K 36/48, A61P 17/16

(54) **Use of legume products for the treatment and prevention of radiotherapy-induced skin damage**
Verwendung von Hülsenfrüchten zur Herstellung von Produkten zum Schutz oder Behandlung von radiotherapie-bedingten Hautschäden
Utilisation de produits à base de légumineuses pour le traitement et la prévention de lésions cutanées induites par radio-thérapie

(30) Priority: 16.07.2004 US 588449 P
(43) Date of publication of application: 19.04.2006
(73) Proprietor: Johnson & Johnson Consumer Companies, Skillman NJ 08558 (US)
(72) Inventor: Seiberg, Miri, Princetown NJ 08540 (US)
(74) Representative: Fisher, Adrian John

(56) References cited:
- EP-A- 1 348 441
- WO-A-01/34099
- US-A- 4 512 973
- US-A1- 2004 062 731
- DATABASE WPI Section Ch, Week 200410 Derwent Publications Ltd., London, GB; Class B04, AN 2004-094136 XP002368309 & JP 2004 000019 A (IKEDA SHOKKEN KK) 8 January 2004 (2004-01-08)

## Description

### FIELD OF THE INVENTION

The present invention relates to the use of non-denatured legume products andtopical compositions containing such non-denatured legume products.

### BACKGROUND OF THE INVENTION

Patients undergoing radiation therapy, or radiotherapy, often experience acute and long-term skin damage ranging from erythema, skin peeling and pain to fibrosis and an increased risk of skin cancer development. These complications may be so severe that they alter the general status and quality of life of such patients. They can result in ulceration, a limited range of motion of the affected area and in transformation into squamous cell carcinoma (Lorette G, Machet L., Radiation-induced skin toxicities: prevention, treatment, Cancer Radiother Acta Oncol, 35 Suppl. 7; 141-8, 1996).

Radiotherapy involves "ionizing" radiation, which is stronger and penetrates more deeply than ultraviolet radiation. It is highly intense and administered in accordance with a specific regimen. Skin changes that are caused by radiation are called radiation dermatitis and are associated with dry or moist desquamation and with an increase in TEWL (Trans-epidermal Water Loss).

Radiotherapy-induced skin lesions of a permanent nature have been demonstrated at both the cellular and the ultrastructural levels (Rudolph R, Arganese T, Woodward M., The ultrastructure and etiology of chronic radiotherapy damage in human skin (Ann Plast Surg, 9:4, 282-92, Oct 1982; Nachtrab U, Oppitz U, Flentje M, Stopper H, Radiation-induced micronucleus formation in human skin fibroblasts of patients showing severe and normal tissue damage after radiotherapy, Int J Radiat Biol 73:3, 279-87, Mar, 1998; Sivan V, Vozenin-Brotons MC, Tricaud Y, Lefaix JL, Cosset JM, Dubray B, Martin MT, Altered proliferation and differentiation of human epidermis in cases of skin fibrosis after radiotherapy, Int J Radiat Oncol Biol Phys 53:2, 385-93, Jun 1, 2002).

There are currently no treatments available for preventing or treating radiotherapy-induced skin damage. Different regimens of fractionated irradiation and post treatment are being evaluated for their ability to reduce radiation-induced skin damage. For example, Van der Schueren et al. (Radiotherapy by multiple fractions per day (MFD) in head and neck cancer: acute reactions of skin and mucosa, Int J Radiat Oncol Biol Phys 19:2, 301-11, Aug, 1990) had evaluated the feasibility of several unusual fractionation schedules in the radiotherapy of head and neck tumors on the acute reactions of skin and mucosa. They concluded that intervals of twelve days permitted full repair of mucosal damage with no cumulative effect. However, as skin damage develops more slowly, even intervals of two weeks were insufficient for the skin to recover fully from radiation damage. Subsequent treatments led to cumulative damage.

Another approach has been to treat patients undergoing radiotherapy for head and neck or breast cancer who had delayed and progressively worse skin reactions with an oral combination of pentoxifylline and tocopherol for at least six months. This approach achieved striking regression of chronic radiotherapy damage. (Delanian S, Balla-Mekias S, Lefaix JL, Striking regression of chronic radiotherapy damage in a clinical trial of combined pentoxifylline and tocopherol, J Clin Oncol 17:10, 3283-90, Oct, 1999). However, this approach cannot be used for prevention of radiation dermatitis.

Irradiation at the correct phase of the circadian cycle was evaluated, but not found useful (Hirn-Stadler B, Rojas A, Influence of circadian rhythms on radiosensitivity: single and fractionated dose studies in mouse skin, Int J Radiat Biol 59:1, 185-93, Jan, 1991).

Topical treatments with various agents have also been evaluated and found to provide only limited response. Hyaluronic acid in lalugen cream was found to reduce the incidence of highgrade radio-epithelitis (Liguori et al., Double-blind, randomized clinical study comparing hyaluronic acid cream to placebo in patients treated with radiotherapy, Radiother Oncol 42:2, 155-61, Feb, 1997).

Another topical product containing a mixture of stearic acid, a hydrophobic component, and propylene glycol, glycerol and polyunsaturated alcohols, hydrophilic components, in a complete treatment rate of 57.9% for acute radiation skin damage, including erythema, dry and moist desquamation and ulceration. Improvement was seen in 36.8% of treated patients and failure in 5.3% of treated patients. (Dini et al., Management of acute radiodermatitis. Pharmacological or nonpharmacological remedies?, Cancer Nurs 16:5, 366-70, Oct, 1993). However, this treatment is not suitable for the prevention of radiation-induced skin damage.

Biafine® cream is a topical preparation used to reduce the risk of, and to treat skin reactions to radiation therapy. This cream provides deep dermal hydration, emollients and stearic acid, and claims to replenish the natural barrier function and the elasticity to intact skin and is indicated for radiation/medical oncology induced redness, erythema, dry/moist desquamation.

Inflammation is mediated, in part, by prostaglandin E (PGE2), which is produced from a reaction catalyzed by the enzymes Cyclooxygenase-1 and 2, (COX-1 and COX-2). Radiation increases PGE2 production, both locally and systemically, contributing to the undesired effects of radiotherapy. Liang and colleagues studied the use of Cox-2 inhibitors in a mouse model system of radiotherapy (Am J Clin Oncol. 2003 Aug;26(4):S114-21). Treatment with the Cox-2 inhibitor celecoxib together with 50 Gy radiation resulted in less inflammation of the dermis compared with saline-treated controls.

Checkpoint kinase-1 (Chk-1) is an essential component of the DNA damage checkpoint. Phosphorylation of Chk1 is required for cells to delay cell cycle progression in response to double-strand DNA breaks, and to enable cells the required time for DNA repair, therefore reducing the risk of cancer. Ionizing radiation induces both the S and the G2 checkpoints, as the cells sense DNA damage and initiate repair mechanisms. Human cells lacking Chk-1 cannot induce S and G2 checkpoints following ionizing irradiation. (Zhao et al., Proc Natl Acad Sci U S A. 2002 Nov 12;99(23):14795-800. Epub 2002 Oct 24). An agent that induces Chk-1 phosphorylation is desired to retain the irradiated cells and tissues in these checkpoints until DNA damage is repaired, to reduce the risk of irradiation-induced cancer.

The foregoing examples relate to addressing the treatment of acute and visible signs of skin damage due to radiation. However, none of these examples examine the ways in which to reduce the risk of radiation-induced skin cancer.

Thus, there is a need for better, safer and more effective agents for the prevention and/or treatment of radiotherapy-induced skin damage.

Legume seeds contain high levels of proteins, lipids and carbohydrates. Consequently, legume seeds, such as soybeans, and compositions containing legume seeds are considered a great nutrient for human use. Legume seeds also contain compounds that inhibit protease activity. For example, two protein protease inhibitors were isolated from soybeans in the early 1940's, the Kunitz-type trypsin inhibitor (soybean trypsin inhibitor, STI) and the Bowman-Birk protease inhibitor (BBI). See, e.g., Birk, Int. J. Pept. Protein Res. 25:113-131 (1985) and Kennedy, Am. J. Clin. Neutr. 68:1406S-1412S (1998).

STI inhibits the proteolytic activity of trypsin by the formation of a stable stoichiometric complex. See, e.g., Liu, K., Chemistry and Nutritional value of soybean components. In: Soybeans, chemistry, technology and utilization. pp. 32-35 (Aspen publishers, Inc., Gaithersburg, MD, 1999). STI consists of 181 amino acid residues with two disulfide bridges and is roughly spherically shaped. See, e.g., Song et al., J. Mol. Biol. 275:347-63 (1998).

BBI is an 8 k-Da protein that inhibits the proteases trypsin and chymotrypsin at separate reactive sites. See, e.g., Billings et al., Pro. Natl. Acad. Sci. 89:3120-3124 (1992). STI and BBI are found only in the soybean seed, and not in any other part of the plant. See, e.g., Birk, Int. J. Pept. Protein Res. 25:113-131 (1985).

### SUMMARY OF THE INVENTION

This invention relates to the use of legume products having trypsin inhibitory activity and reduced microbial content for the manufacture of a medicament for preventing and treating radiotherapy-induced skin damage.

Other features and advantages of the present invention will be apparent from the detailed description of the invention and from the claims.

### DETAILED DESCRIPTION OF THE INVENTION

We believe that one skilled in the art can, based upon the description herein, utilize the present invention to its fullest extent. The following specific embodiments are to be construed as merely illustrative.

Unless defined otherwise, all technical and scientific terms used herein have the same meaning as commonly understood by one of ordinary skill in the art to which the invention belongs. As used herein, all percentages are by weight unless otherwise specified.

As used herein, "trypsin inhibitory activity" means the ability of e.g., the legume product at a concentration of 0.1% (w/w) to inhibit the activity of the protease trypsin, as measured by the assay set forth below in Example 2. Such activity may also be capable of being performed by pure soy-derived agents or by synthetic molecules that have trypsin inhibitory activity. In one embodiment, the legume products of the present invention have a trypsin inhibitory activity of at least about 15%. In a further embodiment, the legume products of the present invention have a trypsin inhibitory activity of at least about 25%, such as at least about 35%.

As used herein, "microbial content" means the amount of bacteria, fungi, and yeast present in the legume product. Examples of means to measure microbial content include, but are not limited to, the AOAC 986.23 Method as set forth in "Official Methods of Analysis of AOAC International," edited by Patricia Cunniff, Sixteenth Edition, 5th Revision, 1999 (AOAC International) or the USP Method as set forth in "Official Compendia of Standards, USP 24 USP/NF 19", United States Pharmacopeial Convention, Inc., 2000 (Board of Trustees, United States Pharmacopeial Convention, Inc.).

"Objectionable microbial content" means the amount of bacteria, fungi, and yeast present in the legume product that are harmful to humans, including but not limited to coliform, E. Coli, Salmonella, thermophilic spores, Bacillus, Enterococcus, Staphylococcus, fecal streptococcus, and those listed in "Disinfection, sterilization, and preservation" 4th edition, Seymour S. Block, pp. 887-888 (1991, Lea & Febiger, Malvern, PA).

As used herein, "topical application" means directly laying on or spreading on skin using, e.g., by use of the hands or an applicator such as a wipe, puff, roller, or spray.

As used herein, "cosmetically-acceptable" means that the product(s) or compound(s) which the term describes are suitable for use in contact with tissues (e.g., the skin) without undue toxicity, incompatibility, instability, irritation, allergic response, and the like.

As used herein, "topical carrier" means one or more compatible solid or liquid filler diluents that are suitable for topical administration to a mammal. Examples of topical carriers include, but are not limited to, water, waxes, oils, emollients, emulsifiers, thickening agents, gelling agents, and mixtures thereof.

As used, herein, "treatment of radiotherapy-induced skin damage" means the reduction or prevention of the damage from ionizing radiation in mucosa, skin, hair, or nails. Examples of radiotherapy-induced skin damage include, but are not limited to, damage to the mucosa, skin, hair, and nails including, but not limited to, dermatitis, erythema, epithelitis, skin peeling, pain, fibrosis, ulceration, lesions and increased risk of skin cancer due to, for example, increased DNA damage. It may also include an increase in TEWL, which we believe is caused by barrier impairment.

As used herein, "safe and effective amount" means an amount of compound or composition (e.g., the legume product) sufficient to induce a positive modification in the condition to be regulated or treated, but low enough to avoid serious side effects. The safe and effective amount of the compound or composition will vary with the particular condition being treated, the age and physical condition of the end user, the severity of the condition being treated/prevented, the duration of the treatment, the nature of other treatments, the specific compound or product/composition employed, the particular cosmetically-acceptable carrier utilized, and like factors.

United States patent applications Serial No. 09/698,454 filed October 27, 2000 and Serial No. 10/689,149, filed October 20, 2003, describe compositions containing non-denatured soy products, or trypsin inhibitors.

### Legume Product

What is meant by a "legume product" is a substance derived from a legume seed. A legume is a plant from the family Leguminosae, which has a dehiscent seed such as a bean, pea, or lentil. Examples of legumes, include but are not limited to, beans such as soybeans, lentil beans, peas, and peanuts.

The legume product may contain the entire legume seed (e.g., the legume seed ground into a powder) or only a portion of the legume (e.g., an extract of the legume). The legume product may be in the form of a fluid (e.g., a mixture of the legume seed and water) or a solid (e.g., legume seeds powders). When in the form of a fluid, the term "legume product" refers to the solid constituents of the fluid derived from the legume.

The compositions for use in the present invention comprise a safe and effective amount of the legume product (e.g., soy product). In one embodiment, the composition contains from about 0.001% to about 50%, from about 1% to about 30%, of the legume product (e.g., soy product).

### Soy Product

The compositions for use in this invention contain legume products, and preferably soy products, that may be in the form of a fluid (e.g., soymilk) or a solid (e.g., a soybean powder, soybean flour, or soymilk powder). What is meant by "soy product" is a substance derived from the soybean, containing the ingredients naturally found in soybeans, at the relative concentrations as found in the beans, with exception of water content. The soy product may contain only a portion of the soybean (e.g., an extract of the soybean such as a lipid reduced soybean powder or filtered soymilk) or may contain the entire soybean (e.g., a ground powder of the legume). In one embodiment, the soy product is a non-denatured soy product. "Denaturation" is defined in the Bantam Medical Dictionary (1990 edition) as "the change in the physical and the physiological properties of a protein, that are brought about by heat, X-rays or chemicals. These changes include loss of activity (in the case of enzymes) and loss (or alteration) of antigenicity (in the case of antigens)". What is meant by "non-denatured soy product" is a soy product in which the processing for the derivation of such soy product (e.g., the temperature, extraction media) did not eliminate its protease inhibitory activity. In one embodiment, the non-denatured state of the soy product of this invention is measured by the presence of an intact soybean trypsin inhibitor (STI) protein. In another embodiment it is measured by the presence of trypsin inhibitory activity as described in example 2.

The soy product may be in the form of a fluid (e.g., soymilk) or a solid (e.g., a soybean powder or soymilk powder). When in the form of a fluid, the term "soy product" refers to the solid constituents of the fluid that are derived from the soybean. In one embodiment, the soy product is soybean powder. Soybean powder may be made by grinding dry soybeans. In one embodiment, the soybean powder has an average particle size of less than about 500 micrometers such as less than about 200 micrometers. In one embodiment, the soybean powder has a moisture content of less than about 10% such as less than about 5%. In one embodiment, the soybean powder is lyophilized.

In one embodiment, the soy product is soymilk or soymilk powder. Soymilk is a combination of solids derived from soybeans and water, the mixture of which has some or all of the insoluble residues filtered off. Soymilk powder is evaporated soymilk, which in one embodiment, is in a lyophilized or spray-dried form. Procedures for manufacturing soymilk include, but are not limited to, the following three procedures. First, soymilk may be made by placing soybeans into water to allow them to absorb the water. The swelled beans are then ground and additional water is then added. The mixture may then filtered to remove any fibrous residue. Second, soymilk may also be prepared from soybean powder. Soybean powder is thoroughly mixed with water (e.g., for at least one hour), which may then be followed by a filtration process to remove fibrous residues. Third, soymilk can also be reconstituted from soymilk powder by adding water. In one embodiment, soymilk comprises from between about 1% to about 50%, by weight (e.g., from about 2.5% to about 20%, by weight) of solids from the soybean.

### Anti-microbial Treatment of Legume Product

As discussed above, the surface of legume seeds often contain high levels of microorganisms. Thus, prior to use by humans, the legume product needs to be treated to reduce or eliminate such microorganisms.

In one embodiment, the legume products for use in the present invention have a total microbial content of less than about 10,000 colony-forming units ("cfu") per gram. In a further embodiment, the soy products of the present invention have a microbial content of less than about 1,000 cfu per gram (such as less than about 100 cfu per gram) of the legume product.

In one embodiment, the legume products for use in the present invention have a total objectionable microbial content of less than 300 cfu per gram such as less than 150 cfu per gram. In a further embodiment, the legume products for use in the present invention have an undetectable amount of any objectionable microbials for at least one gram (e.g., at least ten grams) of legume product.

In one embodiment, the legume product is exposed to gamma irradiation. In a further embodiment, the legume product is exposed to between about 2 to about 30 kGy of gamma irradiation, such as between about 5 and about 10 kGy of gamma irradiation. Applicants have unexpectedly found that such treatment reduces the microbial content of the legume product, while maintaining its biological activity (e.g., serine protease inhibitory activity), as set forth in U.S. Patent No. 6,555,143B2.

Applicants have also found that treatment of legume products with gamma irradiation maintains the cosmetic elegance of the legume product, such as maintained its natural colors and did not induce significant malodors. Other anti-microbial processes that also maintain the protease inhibitory activity of the legume product that can be practiced alone or in combination with gamma irradiation, include, but are not limited to, exposure to x-rays, high energy electron or proton beams, ultraviolet radiation, hydrostatic pressure, and addition of chemical agents possessing antimicrobial activity, and combinations thereof. A complete list of methods for microbial content reduction is set forth in "Disinfection, sterilization, and preservation" 4th edition, Seymour S. Block, pp. 887-888 (1991, Lea & Febiger, Malvern, PA).

Applicants have found that processes using thermal treatment may result in a substantial loss in protease inhibitory activity and, thus, should be used with caution. For example, applicants have found that heating soymilk to 100°C for only 10 minutes reduced the trypsin inhibitory activity of the soymilk from 86% (when maintained at 4°C) to 46%. Applicants have found that heating soymilk can also result in a change of the color or odor of the soybean product.

### Topical Compositions

The topical compositions useful in the present invention involve formulations suitable for topical application to skin. In one embodiment, the composition comprises the soy product and a cosmetically-acceptable topical carrier. In one embodiment, the cosmetically-acceptable topical carrier is from about 50% to abut 99.99%, by weight, of the composition (e.g., from about 80% to about 95%, by weight, of the composition).

The compositions may be made into a wide variety of product types that include but are not limited to lotions, creams, gels, sticks, sprays, shaving creams, ointments, cleansing liquid washes and solid bars, shampoos, pastes, powders, mousses, shaving creams, wipes, patches, nail lacquers, wound dressing and adhesive bandages, hydrogels, films and make-up such as foundations, mascaras, and lipsticks. These product types may comprise several types of cosmetically acceptable topical carriers including, but not limited to solutions, emulsions (e.g., microemulsions and nanoemulsions), gels, solids and liposomes. The following are non-limitative examples of such carriers. Other carriers can be formulated by those of ordinary skills in the art.

The topical compositions useful in the present invention can be formulated as solutions. Solutions typically include an aqueous solvent (e.g., from about 50% to about 99.99% or from about 90% to about 99% of a cosmetically acceptable aqueous solvent).

Topical compositions useful in the subject invention may be formulated as a solution comprising an emollient. Such compositions preferably contain from about 2% to about 50% of an emollient(s). As used herein, "emollients" refer to materials used for the prevention or relief of dryness, as well as for the protection of the skin. A wide variety of suitable emollients are known and may be used herein. Sagarin, Cosmetics, Science and Technology, 2nd Edition, Vol. 1, pp. 32-43 (1972) and the International Cosmetic Ingredient Dictionary and Handbook, eds. Wenninger and McEwen, pp. 1656-61, 1626, and 1654-55 (The Cosmetic, Toiletry, and Fragrance Assoc., Washington, D.C., 7th Edition, 1997) (hereinafter "ICI Handbook") contains numerous examples of suitable materials.

A lotion can be made from such a solution. Lotions typically comprise from about 1% to about 20% (e.g., from about 5% to about 10%) of an emollient(s) and from about 50% to about 90% (e.g., from about 60% to about 80%) of water.

Another type of product that may be formulated from a solution is a cream. A cream typically comprises from about 5% to about 50% (e.g., from about 10% to about 20%) of an emollient(s) and from about 45% to about 85% (e.g., from about 50% to about 75%) of water.

Yet another type of product that may be formulated from a solution is an ointment. An ointment may comprise a simple base of animal or vegetable oils or semi-solid hydrocarbons. An ointment may comprise from about 2% to about 10% of an emollient(s) plus from about 0.1% to about 2% of a thickening agent(s). A more complete disclosure of thickening agents or viscosity increasing agents useful herein can be found in Sagarin, Cosmetics, Science and Technology, 2nd Edition, Vol. 1, pp. 72-73 (1972) and the ICI Handbook pp. 1693-1697.

The topical compositions useful in the present invention formulated as emulsions. If the carrier is an emulsion, from about 1% to about 10% (e.g., from about 2% to about 5%) of the carrier comprises an emulsifier(s). Emulsifiers may be nonionic, anionic or cationic. Suitable emulsifiers are disclosed in, for example, U.S. Patent No. 3,755,560, U.S. Patent No. 4,421,769, McCutcheon's Detergents and Emulsifiers, North American Edition, pp. 317-324 (1986), and the ICI Handbook, pp.1673-1686.

Lotions and creams can be formulated as emulsions. Typically such lotions comprise from 0.5% to about 5% of an emulsifier(s). Such creams would typically comprise from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

The compositions for use in this invention should contain an amount of non-denatured soy to be effective in protecting, preventing and/or treating radiation-induced skin damage. There should not be so much soy present in the formulation so as to produce a grainy or unaesthetic composition. Preferably, the compositions for use in this invention should contain non-denatured soy in an amount from about 0.1 to about 15% by weight of the composition. More preferably, they should contain non-denatured soy in an amount from about 0.5 to about 10% by weight of the composition. Most preferably, they should contain non-denatured soy in an amount from about 2 to about 6% by weight of the composition.

Single emulsion skin care preparations, such as lotions and creams, of the oil-in-water type and water-in-oil type are well-known in the cosmetic art and are useful in the subject invention. Multiphase emulsion compositions, such as the water-in-oil-in-water type, as disclosed in U.S. Patent No. 4,254,105 and 4,960,764, are also useful in the subject invention. In general, such single or multiphase emulsions contain water, emollients, and emulsifiers as essential ingredients.

The topical compositions for use in this invention can also be formulated as a gel (e.g., an aqueous gel using a suitable gelling agent(s)). Suitable gelling agents for aqueous gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically comprises between about 0.1% and 5%, by weight, of such gelling agents.

The topical compositions for use in the present invention can also be formulated into a solid formulation (e.g., a wax-based stick, soap bar composition, powder, or a wipe containing powder).

Liposomal formulations are also compositions useful in the subject invention. Examples of liposomes are unilamellar, multilamellar, and paucilamellar liposomes, which may or may not contain phospholipids. Such compositions can be prepared by first combining hesperetin with a phospholipid, such as dipalmitoylphosphatidyl choline, cholesterol and water according to the method described in Mezei & Gulasekharam, "Liposomes--A Selective Drug Delivery System for the Topical Route of Administration; Gel Dosage Form", Journal of Pharmaceutics and Pharmacology, Vol. 34 (1982), pp. 473-474, or a modification thereof. Epidermal lipids of suitable composition for forming liposomes may be substituted for the phospholipid. The liposome preparation may then be incorporated into one of the above carriers (e.g., a gel or an oil-in-water emulsion) in order to produce the liposomal formulation. Other compositions and pharmaceutical uses of topically applied liposomes are described in Mezei, M., "Liposomes as a Skin Drug Delivery System", Topics in Pharmaceutical Sciences (D. D. Breimer and P. Speiser, eds.,), Elsevier Science Publishers B. V., New York, N.Y., 1985, pp. 345-358, PCT Patent Application No. WO96/31194 and U.S. Patent No. 5,260,065.

The topical compositions useful in the subject invention may contain, in addition to the aforementioned components, a wide variety of additional oil-soluble materials and/or water-soluble materials conventionally used in compositions for use on skin, hair, and nails at their art-established levels.

### Additional Cosmetically and Pharmacologically Active Agents

In one embodiment, the topical composition further comprises another cosmetically or pharmaceutically active agent in addition to the soy product. What is meant by a "cosmetically or pharmaceutically active agent" is a compound (e.g., a synthetic compound or a compound or a mixture of compounds, either synthetic isolated from a natural source) that has a cosmetic or therapeutic effect on the skin, hair, or nails, including, but not limiting to e.g., lightening agents, darkening agents such as self-tanning agents, anti-acne agents, anti-cancer agents, cancer-preventing agents, shine control agents, anti-microbial agents, anti-inflammatory agents, anti-mycotic agents, anti-parasite agents, external analgesics, sunscreens, photoprotectors, antioxidants, keratolytic agents, detergents/surfactants, moisturizers, nutrients, vitamins, energy enhancers, anti-perspiration agents, astringents, deodorants, hair removers, hair growth-inducing agents, firming agents, anti-callous agents, and agents for hair, nail, and/or skin conditioning.

Natural extracts containing antioxidants suitable for use in the compositions of this invention, include, but not limited to, extracts containing flavonoids and isoflavonoids and their derivatives (e.g., genistein and daidzein), extracts containing resveratrol and the like. Examples of such natural extracts include grape seed, green tea, pine bark, feverfew and propolis. Other examples of antioxidants may be found on pages 1612-13 of the ICI Handbook. Other extracts from natural sources containing pigments (e.g., brown pigments from plants from the Hedychium genus or Bearberry genus or yellow, orange and red pigments, from plants containing carotenoids or canthaxanthins) may also be employed in the topical compositions for use in this invention.

### Mineral Water

The compositions for use in the present invention may be prepared using a mineral water, for example mineral water that has been naturally mineralized such as Evian® Mineral Water (Evian, France). In one embodiment, the mineral water has a mineralization of at least about 200 mg/L (e.g., from about 300 mg/L to about 1000 mg/L). In one embodiment, the mineral water comprises at least about 10 mg/L of calcium and/or at least about 5 mg/L of magnesium.

### Anti-inflammatory Agents

The compositions for use in this invention may also contain anti-inflammatory agents that are synthetic or naturally-derived, including corticosteroids such as hydrocortisone and the like, non-steroidal anti-inflammatory drugs, including ibuprofen, salicylates, naproxen salts, acetominophen, COX-2 inhibitors and the like, feverfew and other anti-inflammatory compounds or extracts that are naturally-derived.

In one embodiment, the agent is selected from, but not limited to, the group consisting of hydroxy acids, benzoyl peroxide, sulfur resorcinol, ascorbic acid, D-panthenol, hydroquinone, octyl methoxycinnimate, titanium dioxide, octyl salicylate, homosalate, avobenzone, polyphenolics, carotenoids, free radical scavengers, spin traps, retinoids such as retinol and retinyl palmitate, ceramides, polyunsaturated fatty acids, essential fatty acids, enzymes, enzyme inhibitors, minerals, hormones such as estrogens, steroids such as hydrocortisone, 2-dimethylaminoethanol, copper salts such as copper chloride, peptides containing copper such as Cu:Gly-His-Lys, coenzyme Q10, peptides such as those disclosed in PCT Patent Application WO00/15188, lipoic acid, amino acids such a proline and tyrosine, vitamins and their related molecules such as retinoic acid, retinol, alpha, gamma or delta tocopherols and other related tocopherols or their mixture, lactobionic acid, acetyl-coenzyme A, niacin, riboflavin, thiamin, ribose, electron transporters such as NADH and FADH2, and other botanical extracts such as aloe vera, feverfew, Bugrane-P, and derivatives and mixtures thereof. The cosmetically active agent will typically be present in the composition for use in the invention in an amount of from about 0.001% to about 20% by weight of the composition, e.g., about 0.01% to about 10% such as about 0.1% to about 5%.

Examples of vitamins include, but are not limited to, vitamin A, Vitamin A precursors such as retinol or retinol esters, vitamin Bs such as vitamin B3, vitamin B5, and vitamin B12, vitamin C, vitamin K, and vitamin E such as alpha, gamma or delta tocopherol and other related tocopherols or tocopherol mixtures.

Examples of hydroxy acids include, but are not limited, to glycolic acid, lactic acid, malic acid, salicylic acid, citric acid, and tartaric acid. See, e.g., European Patent Application No. 273,202.

Examples of antioxidants include, but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, and ascorbic acid and ascorbic acid derivatives (e.g., ascorbyl palmitate and ascorbyl polypeptide). Oil-soluble antioxidants suitable for use in the compositions of this invention include, but are not limited to, butylated hydroxytoluene, retinoids (e.g., retinol and retinyl palmitate), different tocopherols (e.g., alpha, beta, gamma, delta and their mixtures, e.g. in the form of tocopherol acetate), tocotrienols, and ubiquinone. Natural extracts containing antioxidants suitable for use in the compositions of this invention, include, but not limited to, extracts containing flavonoids and isoflavonoids and their derivatives (e.g., genistein and daidzein), extracts containing resveratrol and the like. Examples of such natural extracts include grape seed, green tea, pine bark, and propolis. Other examples of antioxidants may be found on pages 1612-13 of the ICI Handbook.

### Other Materials

Various other materials may also be present in the compositions useful in the subject invention. These include humectants, proteins and polypeptides, preservatives and an alkaline agent. Examples of such agents are disclosed in the ICI Handbook, pp.1650-1667.

The compositions for use in the present invention may also comprise chelating agents (e.g., EDTA) and preservatives (e.g., parabens). Examples of suitable preservatives and chelating agents are listed in pp. 1626 and 1654-55 of the ICI Handbook. In addition, the topical compositions useful herein can contain conventional cosmetic adjuvants, such as dyes, opacifiers (e.g., titanium dioxide), pigments, and fragrances.

### Regimen

Preferably, the compositions for use in this invention would be applied to the area of a patient's skin that would be exposed to radiation during the course of radiotherapy ("treatment area"). More preferably, the compositions of this invention would be applied to the treatment area prior to exposure to radiation and subsequent to each radiotherapy session. Most preferably, the compositions of this invention would be applied to the treatment area once daily for at least one day and preferably 1-2 weeks prior to exposure to radiation, subsequent to each radiotherapy session and once daily for at least four weeks subsequent to the completion of radiotherapy.

The composition and formulations containing such compositions for use in the present invention may be prepared using methodology that is well known by an artisan of ordinary skill.

### Example 1: Gamma Irradiation of Legume Product

Applicants have found that soymilk powder prior to any antimicrobial processing such as gamma irradiation has high levels microbial content, ranging from up to 50,000 cfu per gram. Such products were also found to have detectable levels of objectionable microbial content, such as fecal streptococci, at levels up to 20,000 cfu per gram.

Applicants have exposed various amounts (e.g., from about 1 g to about 200 kg) of soymilk powder to gamma irradiation varying from 1 kGy to 16 kGy. The dose or gamma irradiation needed for a reduction a total microbial content to less than about 100 cfu per gram was found to be about 10 kGy. The dose for one log reduction for fecal streptococci is determined to be about 3 kGy and a dose of about 5 kGy was found to consistently reduce this microbial content within a 10 gram sample of soymilk powder to undetectable levels. However, the amount of gamma irradiation used on the legume product will ultimately be determined by the microbial content and size of the soy product to be so treated.

### Example 2: Trypsin Inhibitory Activity of Legume Product

The inhibition of trypsin-induced cleavage of a fluorescent casein peptide was measured using the EnzChek™ protease assay kit, following manufacturer's instructions (EnzChek™ Protease Assay Kits Product Information, Revised 3/15/99; Molecular Probes, Eugene OR). In summary, various soy preparations were first diluted in 1X digestion buffer (provided in kit) and incubated at different concentrations with 100 units of trypsin (Sigma, St. Louis, MO) dissolved in 1X digestion buffer. A pure trypsin inhibitor (soybean trypsin inhibitor, from Sigma, St. Louis, MO) was used as a positive control at 0.1, 0.01%, and 0.001% w/v. Then, 1.0 mg/ml stock solution of BODIPY FL casein was prepared by adding 0.2 mL of deionized water to the vials supplied with this substrate (provided in kit), then made to a final working concentration of 10 microgram/ml in digestion buffer. Following incubation of the trypsin, with or without the test material, with the BODIPY fluorescent casein substrate at room temperature for one hour, fluorescence was measured (excitation 485 nm /emission 530 nm) on a SpectraMax® Gemini microtiter plate reader (Molecular Devices Corporation, Sunnyvale, CA) using Softmax® Pro 3.0 software (Molecular Devices Corporation). Each experiment was performed in three replicates and was repeated twice.

This assay was performed on soy products processed seven different ways. Example A was soybeans ground into powder (Sunlight Foods Corporation, Taipei County, Taiwan, R.O.C.). Example B was soybean powder of Example A exposed to about 8-15 kGy of gamma irradiation. Example C was soybean powder in which the oil in the soybean powder was removed by extraction (Soyafluff® 200W from Central Soya Company, Inc., Fort Wayne, IN). Example D was soymilk powder made with dehulled soybeans and water that was subsequently filtered and heated and spray dried (Devansoy Farms, Carroll, Iowa) and exposed to between about 7 - 9 kGy of gamma irradiation. Example E was soymilk powder obtained by mixing soy beans and water, heating the mixture overnight, and adding 1,3-butylene glycol to the mixture (Flavosterone SB from Ichimaru Pharcos Co., Ltd, Gifu Japan). Example F was soymilk powder obtained by mixing soy beans and water, heating the mixture overnight, and subsequently adding ethanol to the mixture (Flavosterone SE from Ichimaru Pharcos Co., Ltd, Gifu Japan). Example G was an extract of soy proteins (Vegeseryl HGP LS 8572 from Laboratories Serobiologiques S.A., Pulnoy, France). These soy products were compared to Soy Trypsin Inhibitor (STI) (Sigma).

The percent inhibition of trypsin cleavage of the substrate by the different soy preparations was calculated using Microsoft Excel™ and is reported in Table 1.

**TABLE 1**

| Tested Product | Concentration (%) | % Inhibition of Trypsin |
|---|---|---|
| STI | 0.01 | 43.0 |
| STI | 0.1 | 76.1 |
| Example A | 0.01 | 32.8 |
| Example A | 0.1 | 67.1 |
| Example B | 0.01 | 31.5 |
| Example B | 0.1 | 67.2 |
| Example C | 0.01 | 22.7 |
| Example C | 0.1 | 36.2 |
| Example D | 0.01 | 8.92 |
| Example D | 0.1 | 17.4 |
| Example E | 0.01 | 7.83 |
| Example E | 0.1 | 10.8 |
| Example F | 0.01 | 4.87 |
| Example F | 0.1 | 5.99 |
| Example G | 0.1 | 6.85 |

As shown in Table 1, STI can inhibit trypsin-induced cleavage in a dose response manner. Example A, which is soybean powder, also significantly inhibited trypsin activity. Further gamma irradiation of the soybean powder (i.e., Example B), while reducing the microbial content of the soybean powder, unexpectedly did not significantly impact the trypsin inhibition activity of the soybean powder. The heat and/or extraction processing of Examples C-G, however, did significantly reduce the trypsin inhibitory activity of the soybean powder.

### Example 3: Prevention of Cox-2 upregulation

Radiation is known to increase PGE2 production, both locally and systemically, via the upregulation of Cox-2 expression. This Cox-2 upregulation contributes to the undesired side effects of radiotherapy, and drugs that inhibit Cox-2 were shown to reduce these undesired effects. We unexpectedly found that Non-denatured Soy extracts have Cox-2 inhibitory activity by decreasing Cox-2 expression.

Non-GMO soybeans (from Devansoy, Carroll, Iowa, were grinded to Enzact soybean powder by Natural Products, Inc. (NPI, Grinnel, Iowa), and γ-irradiated by Isomedix Inc. (Mentor, Ohio). Soy powder (2%) was suspended in Phosphate-buffered saline (PBS, from Invitrogen, Carlsbad, CA) and sonicated on ice 3x for 10 seconds using a Sonics Vibra-cell Sonicator (from Sonics Corp., Newton, CT).

HaCaT cells (a human keratinocyte cell line) were seeded onto 24-well culture plates (-2 x 10⁴ cells/well) and grown in DMEM+10% Fetal bovine serum (Invitrogen Corp., Carlsbad, CA) at 37°C, 5% CO₂ for 24 hours. The cells were then serum starved for 24 hours in DMEM. HaCaT cells were then treated with soy samples in DMEM in triplicate for 24 hours. UVB irradiation (30 mJ/cm²) was performed with a UVB FS light source (Spectronics Corp. Westbury, NY, Ble-1T156 light bulb) in an exposure chamber, with plate covers removed and PBS present in the wells. UVB intensity was measured with 1 UVX radiometer (UVP Inc., San Gabrial, CA). After irradiation, PBS was immediately replaced with DMEM medium. The cells not exposed to UVB were exposed to the same procedure except without the UVB irradiation. At 6 hours post UVB irradiation, cells were lysed in 100 µl of RNA lysis buffer from Ambion (Austin, TX) for RNA extraction. RNA was extracted with RNAqueous kit from Ambion (Austin, TX) according to manufacturer's instructions. Fifty nanograms of total RNA from each sample were subjected to one step RT-PCR reaction using OneStep RT-PCR Kit (QIAGEN® , Valencia, CA) according to manufacturer's instructions. The reverse transcription was carried out for 30 minutes at 50°C and a hot start of 15 minutes at 95°C was then included to activate HotStarTaq™ DNA polymerase in the reaction mix. The reaction contained 30 pmol of each primer of COX-2 (COX-2 sense primer: TGA AAC CCA CTC CAA ACA CA; COX-2 antisense primer: CAG CAA ACC GTA GAT GCT CA), and 10 pmol of each primer of glyceraldehydes-3-phospoate dehydrogenase (GAPDH sense primer: ACC ACA GTC CAT GCC ATC AC; GAPDH antisense primer: TCC ACC ACC CTG TTG CTG TA). PCR consisted of 33 cycles for COX-2 and 23 cycles of GAPDH of denaturing for 50 seconds at 94°C, annealing for 1 minute at 58°C, polymerization for 1 minute at 72°C. The final extension step consisted of 10 minutes at 72°C. The PCR products were resolved by electrophoresis in 1.5% agarose gel and stained with ethidium bromide. Gel images of RT-PCR products were analyzed by Kodak Gel Logic 100 Imaging System (Eastman Kodak Co., New Haven, CT). The results are presented in Table 2 below as the ratio of Cox-2 expression to a GAPDH housekeeping gene control.

**TABLE 2**

| Treatment | Cox-2 mRNA level (ratio to GAPDH control) |
|---|---|
| Untreated | 1 |
| 0.1% Soy | 0.03 |
| UVB | 1.98 |
| 0.1% Soy + UVB | 0.51 |

These data demonstrate that Soy reduces both the basal level and the UVB-induced level of Cox-2 expression, suggesting that the non-denatured Soy extract has a Cox-2 inhibitory activity. The down regulation of Cox-2 activity should reduce undesired inflammatory processes induced by radiotheray treatment.

### Example 4: Activation of Chk-1

In response to double-strand DNA damage, which could be induced by different types of irradiation, including UV and ionizing irradiation, cells induce Chk-1 phosphorylation. Chk-1 activation delays the progression of the cell cycle, and enables cells the required time for DNA repair, therefore reducing the risk of cancer. We unexpectedly found that non-denatured Soy extracts induce Chk-1 phosphorylation, and the induction is significantly higher in the presence of DNA damage.

HaCaT cells were plated and Soy-treated as indicated in Example 3, with or without UVB exposure. Cells were harvested 1 hour after UVB, washed with ice-cold PBS containing 50mM NaF and 1mM Sodium Orthovanidate (Sigma, St. Louis, MO). RIPA buffer (200 µl) (65 mM TRIS, 150mM NaCl, 1% NP-40, 0.25% Sodium Deoxycholate, 1 mM EDTA, pH 7.4 (from Sigma, St. Louis, MO), containing the above phosphatase inhibitors and a Complete Mini Protease Inhibitor Cocktail (Roche Applied Sciences, Indianapolis, IN), was added and the cells were mechanically dissociated using a cell scraper. The lysates were frozen and stored at -20°C.

The samples were centrifuged at 14,000rpm for 10 minutes at 4°C to pellet insoluble material. Total protein was then assessed using the BioRad D_{c} Protein Assay (BioRad Laboratories, Inc., Hercules, CA). The samples were then mixed 1:1 with Laemmli sample buffer containing β-mercaptoethanol, boiled for 5 minutes, and centrifuged. The samples were normalized for total protein and then loaded on 4-20% Tris-Glycine minigels (Invitrogen Corp., Carlsbad, CA). The proteins were transferred to PVDF membrane (BioRad Laboratories, Inc., Hercules, CA), blocked with 10% milk (from BioRad Laboratories, Inc., Hercules, CA) and probed overnight with antibodies for Chk and phospho-Chk (all obtained from Cell Signaling Technology, Inc., Beverly, MA). Antibodies for Chk-2 were used for control. The blots were washed with PBST (1x PBS with 0.2% Tween-20 (Sigma, St. Louis, MO)), probed with an anti-Rabbit:HRP conjugated secondary antibody (Invitrogen Corp., Carlsbad, CA) in PBST and 5% milk for 1 hour, then washed 3x with PBST. Proteins were visualized by incubating the blots in ECL Plus reagent (Amersham Biosciences, Piscataway, NJ) for 1 minute, draining the excess liquid and exposing to Hyperfilm ECL (Amersham Biosciences, Piscataway, NJ) for various lengths of time. The film was developed using a Konica SRX-101A Processor (Pacific Northwest X-Ray Inc., Gresham, OR). The results were captured electronically and densitometry quantified using a Kodak Gel Logic 100 Imaging System and Kodak 1_{D} imaging software (Eastman Kodak Co., New Haven, CT). Table 3 below describes the results as the ratio of Chk-1 phosphorylation to total Chk-1. Since the level of phosphorylated Chk-1 is barely detected in cells free of DNA damage, this ratio is defined as TLTD (too low to be determined). A TLTD ratio suggests very low level of Chk-1 activation. Similar studies for Chk-2 are presented as a control.

**TABLE 3**

| Treatment | pChk-1:Chk-1 | pChk-2:Chk-2 |
|---|---|---|
| Untreated | TLTD | 1.0 |
| Soy 0.05% | TLTD | 1.05 |
| Soy 0.1% | TLTD | 1.29 |
| UVB | 1.00 | 2.24 |
| Soy 0.05% + UVB | 2.33 | 2.24 |
| Soy 0.1% + UVB | 4.23 | 1.49 |

These data document an increase in Chk-1 activation in the presence of the Soy extract, when DNA damage is induced, enabling a better DNA repair and reducing the risk of cancer.

### Example 5: Prevention of Radiotherapy-Induced Skin Damage

Non-denatured soybean formulations containing glycerine, chelating agents, emollients, surfactants, skin conditioning agents, an antioxidant, soymilk powder (5% by weight concentration), preservatives and skin and hair conditioning agents were applied to the shaved hind leg skin of C3Hf/Kam males at 12 weeks of age, in a cream emulsion, twice daily. Each of these two daily applications was six hours apart, continuing for ten days prior to irradiation and for five days subsequent to radiation. A control group of mice received placebo treatment. The hind legs were irradiated (XRT) on day 11 (42, 45 or 48 Gy), applying the treatment 3h before XRT using a small animal ¹³⁷Cs irradiator at a dose rate of 5.40 Gy/min. Mice were restrained (without anesthesia) on specially made jigs so that the right rear leg is centered in the 3 cm diameter radiation field. Several mice were sacrificed at 1 and 24 hr post XRT, and skins were processed for histology. The rest of the mice were observed daily for acute skin reactions, ten and about thirty-five days following irradiation. Grading of skin condition was performed using graded qualitative score of response ranging from erythema, dry desquamation, to varying degrees of moist desquamation. The degree of hair loss was also assessed.

The test groups were exposed to single radiation doses of 42 Gy, 45 Gy and 48 Gy, with either none, placebo or Soy treatments. Ninety mice were used for the radiation dose response study and 72 mice were used for histology.

Because the study was not performed with product-level quality, but with a bench-mixed cream, the consistency of the test material was rough and unequal. This was evidenced by the mechanical trauma of applying both the soy and the vehicle control on the animals. Therefore, the most appropriate control for the study is the vehicle plus radiation, not radiation only.

Results of the first part of the study set forth in this Example, the evaluation of skin reaction, show that at doses of 42Gy and 45Gy the soy extract was protective relative to the vehicle control. The reaction curves are shifted to the right to longer times to the peak reaction, and desquamation scores are slightly lower than those in the vehicle treated groups. These results are presented in Tables 4 and 5 below.

The scores for acute skin reaction represent:

| | |
|---|---|
| **0.5** | erythema (reddening) |
| **1.0** | dry desquamation (scaly appearance) |
| **1.5** | focal moist desquamation |
| **2.0** | multiple areas of moist desquamation |
| **2.5** | moist desquamation of half of the treated surface of the leg |
| **3.0** | moist desquamation of more than half of the leg |
| **3.5** | complete exudative desquamation of the entire radiation field of exposure |

The following data suggest that the Soy extract could reduce the skin reaction induced by ionizing irradiation.

**TABLE 4 RADIATION-INDUCED SKIN REACTIONS**

| **Treatment groups*** | **Peak Skin Reaction**** | **Time to Peak (days):** | |
|---|---|---|---|
| | | from 1st treatment | from XRT |
| | Mean (SE) | Mean (SE) | Mean (SE) |
| Shaved only 42 Gy | 1.9 (0.2) | 18.0 (0.8) | 18.0 (0.8) |
| Vehicle only 42 Gy | 3.5 (0) | 21.5 (0.3) | 12.5 (0.3) |
| SOY 42 Gy | 3.0 (0.1) | 21.8 (0.5) | 12.8 (0.5) |
| | | | |
| Shaved only 45 Gy | 2.5 (0.2) | 20.2 (0.6) | 20.2 (0.6) |
| Vehicle only 45 Gy | 3.2 (0.1) | 21.4 (0.4) | 12.4 (0.4) |
| SOY 45 Gy | 2.8 (0.2) | 23.3 (0.4) | 14.3 (0.4) |
| | | | |
| Shaved only 48 Gy | 2.5 (0.1) | 18.3 (0.3) | 18.3 (0.3) |
| Vehicle only 48Gy | 3.2 (0.1) | 21.2 (0.5) | 12.2 (0.5) |
| SOY 48Gy | 3.2 (0.1) | 22.2 (0.5) | 13.2 (0.5) |

| | | | |
|---|---|---|---|
| * Mouse legs were shaved and two days later application of agents began. Vehicle and soy were applied twice daily for a total of 15 days. Legs were irradiated 9 days after the initial application. ** Legs were scored daily for skin reaction and the maximum (Peak) value determined. Skin reaction scores are defined as set forth above | | | |

**TABLE 5**

| **RADIATION-INDUCED SKIN REACTIONS** | | |
|---|---|---|
| **Treatment goups*** | **Time to Heal (days)**** | |
| | from XRT | from Peak |
| | Mean (SE) | Mean (SE) |
| Shaved only 42 Gy | 23.5 (0.8) | 5.3 (1.2) |
| Vehicle only 42 Gy | 23.7 (1.3) | 11.2 (1.3) |
| SOY 42 Gy | 22.7 (0.7) | 9.9 (0.9) |
| | | |
| Shaved only 45 Gy | 27.0 (0.9) | 6.8 (0.7) |
| Vehicle only 45 Gy | 23.4 (0.8) | 11.0 (1.0) |
| SOY 45 Gy | 23.1 (1.3) | 8.8 (1.3) |
| | | |
| Shaved only 48 Gy | 25.5 (1.3) | 7.2 (1.1) |
| Vehicle only 48Gy | 24.9 (1.6) | 13.7 (2.1) |
| SOY 48Gy | 24.1 (1.3) | 10.9 (1.5) |

| | | |
|---|---|---|
| * Mouse legs were shaved and two days later application of agents began. Vehicle and soy were applied twice daily for a total of 15 days. Legs were irradiated 9 days after 1st application. **Healed skin reaction is defined as having no moist desquamation (i.e., less than 1.5 score). | | |

It is understood that while the invention has been described in conjunction with the detailed description thereof, that the foregoing description is intended to illustrate the scope of the invention, which is defined by the scope of the appended claims. Other aspects, advantages, and modifications are within the claims.

## Claims

1. The use of a non-denatured legume product for the manufacture of a medicament for treatment or prevention of radiotherapy-induced damage to the skin, wherein said medicament is for application to an area of a patience skin that will be irradiated or has been irradiated with ionizing radiation pursuant to said radiation therapy.

2. The use according to claim 1 wherein said legume product is a non-denatured soy product.

3. The use according to claim 1 wherein said medicament is for application prior to exposure to radiation.

4. The use according to claim 1 wherein said medicament is for application prior and subsequent to exposure to radiation.

5. The use according to claim 3 wherein said medicament is for application daily for from 1 to 14 days prior to exposure to radiation

6. The use according to claim 5 wherein said medicament is for application twice daily at least six hours apart.

7. The use according to claim 4 wherein said medicament ia for application for from 1 to 14 days prior to exposure to radiation and for at least 30 days subsequent to radiation exposure.

8. The use according to claim 1 wherein said medicament is for application subsequent to radiation exposure.

9. The use according to claim 8 wherein said medicament is for application for at least about 30 days subsequent to radiation exposure.

10. The use according to claim 8 wherein said medicament is for application subsequent to each radiation exposure.

11. The-use according to claim 10 wherein said medicament is first applied for from 1 to 14 days prior to a first radiation exposure.

12. The use according to claim 11 wherein said medicament is further applied for at least 30 days subsequent to completion of radiation exposure.

13. The use according to claim 2 wherein said non-denatured soy product is selected from the group consisting of: soybean powder, soymilk, soymilk powder, limabean powder, limabean milk, limabean milk powder, blackbean powder, blackbean milk, blackbean milk powder, and combinations thereof.

14. The use according to claim 13, wherein said non-denatured soy product is present in the composition in an amount effective to prevent radiotherapy-induced akin damage.

15. The use according to claim 14, wherein the amount of non-denatured soy product present in the composition is from about 0.1 to about 20% by weight of the composition.

16. The use according to claim 15, wherein the amount of non-denatured soy product present in the composition is from about 0.5 to about 15% by weight of the composition.

17. The use according to claim 16, wherein the amount of non-denatured soy product present in the composition is from about 1% to about 10% by weight of the composition.

18. The use according to claim 13, wherein said non denatured soy product is present in the composition in an amount affective to treat radiotherapy-induced skin damage

19. The use according to claim 18, wherein the amount of non-denatured soy product present in the composition is from about 0.1 to about 20% by weight of the composition.

20. The use according to claim 19, wherein the amount of non-denatured soy product present in the composition is from about 0-5 to about 15% by weight of the composition..

21. The use according to claim 19, wherein the amount of non-denatured soy product present in the composition is from about 1% to about 10% by weight of the composition.

22. The use according to claim 1 wherein said composition is in the form of an emulsion.

23. The use according to claim 2 wherein said composition is in the form of an emulsion.

24. The use according to claim 2 wherein said composition further comprises an emollient.

25. The use according to claim 2 wherein said composition further comprises a humectant.

26. The use according to clam 13, wherein said composition further comprises one or more of the group consisting of: lightening agents, darkening agents such as self-tanning agents, anti-acne agents, shine control agents, anti-microbial agents, anti-inflammatory agents, anti-mycotic agents, anti-Parasite-agents, external analgesics, sunscreens, photoprotectors, antioxidants, keratolytic agents, detergents/surfactants moisturizers; nutrients, vitamins, energy enhancers, anti-perspiration agents, astringents, deodorants, hair removers, hair-growth inducers firming agents, anti-callous agents, vitamins, bleaching agents, depigmentation agents, foaming agents, conditioners, humectants, fragrances, anti-aging agents, anti-cancer agents, cancer preventing agents and agents for hair, nail and skin conditioning.

## Patentansprüche

1. Verwendung eines nicht-denaturierten Hülsenfruchtproduktes zur Herstellung eines Arzneimittels zur Behandlung oder Verhinderung von durch Strahlentherapie induzierten Schäden an der Haut, wobei das Arzneimittel zum Aufbringen auf eine Fläche der Haut eines Patienten gedacht ist, die aufgrund der Strahlentherapie mit ionisierender Strahlung bestrahlt werden wird oder bestrahlt worden ist.

2. Verwendung nach Anspruch 1, wobei das Hülsenfruchtprodukt ein nicht-denaturiertes Sojaprodukt ist.

3. Verwendung nach Anspruch 1, wobei das Arzneimittel zum Aufbringen vor der Bestrahlung gedacht ist.

4. Verwendung nach Anspruch 1, wobei das Arzneimittel zum Aufbringen vor der und im Anschluss an die Bestrahlung gedacht ist.

5. Verwendung nach Anspruch 3, wobei das Arzneimittel zur täglichen Anwendung für von 1 bis 14 Tage vor der Bestrahlung gedacht ist.

6. Verwendung nach Anspruch 5, wobei das Arzneimittel zum Aufbringen zweimal täglich mit einem Abstand von wenigstens 6 Stunden gedacht ist.

7. Verwendung nach Anspruch 4, wobei das Arzneimittel zum Aufbringen für von 1 bis 14 Tage vor der Bestrahlung und für wenigstens 30 Tage im Anschluss an die Bestrahlung gedacht ist.

8. Verwendung nach Anspruch 1, wobei das Arzneimittel zum Aufbringen im Anschluss an die Bestrahlung gedacht ist.

9. Verwendung nach Anspruch 8, wobei das Arzneimittel zum Aufbringen für wenigstens etwa 30 Tage im Anschluss an die Bestrahlung gedacht ist.

10. Verwendung nach Anspruch 8, wobei das Arzneimittel zum Aufbringen im Anschluss an jede Bestrahlung gedacht ist.

11. Verwendung nach Anspruch 10, wobei das Arzneimittel zunächst für von 1 bis 14 Tage vor einer ersten Bestrahlung aufgebracht wird.

12. Verwendung nach Anspruch 11, wobei das Arzneimittel weiter für wenigstens 30 Tage im Anschluss an die Beendigung der Bestrahlung aufgebracht wird.

13. Verwendung nach Anspruch 2, wobei das nicht-denaturierte Sojaprodukt ausgewählt ist aus der Gruppe, bestehend aus: Sojabohnenpulver, Sojamilch, Sojamilchpulver, Limabohnenpulver, Limabohnenmilch, Limabohnenmilchpulver, Schwarzbohnenpulver, Schwarzbohnenmilch, Schwarzbohnenmilchpulver und Kombinationen davon.

14. Verwendung nach Anspruch 13, wobei das nicht-denaturierte Sojaprodukt in der Zusammensetzung in einer Menge vorhanden ist, die darin wirksam ist, durch Strahlentherapie induzierte Hautschäden zu verhindern.

15. Verwendung nach Anspruch 15, wobei die Menge an nicht-denaturiertem Sojaprodukt, die in der Zusammensetzung vorliegt, von etwa 0,1 bis etwa 20 Gew.-% der Zusammensetzung beträgt.

16. Verwendung nach Anspruch 15, wobei die Menge an nicht-denaturiertem Sojaprodukt, die in der Zusammensetzung vorliegt, von etwa 0,5 bis etwa 15 Gew.-% der Zusammensetzung beträgt.

17. Verwendung nach Anspruch 16, wobei die Menge an nicht-denaturiertem Sojaprodukt, die in der Zusammensetzung vorliegt, von etwa 1 bis etwa 10 Gew.-% der Zusammensetzung beträgt.

18. Verwendung nach Anspruch 13, wobei das nicht-denaturierte Sojaprodukt in der Zusammensetzung in einer Menge vorliegt, die darin wirksam ist, durch Strahlentherapie induzierte Hautschäden zu behandeln.

19. Verwendung nach Anspruch 18, wobei die Menge an nicht-denaturiertem Sojaprodukt, die in der Zusammensetzung vorliegt, von etwa 0,1 bis etwa 20 Gew.-% beträgt.

20. Verwendung nach Anspruch 19, wobei die Menge an nicht-denaturiertem Sojaprodukt, die in der Zusammensetzung vorliegt, von etwa 0,5 bis etwa 15 Gew.-% der Zusammensetzung beträgt.

21. Verwendung nach Anspruch 20, wobei die Menge an nicht-denaturiertem Sojaprodukt, die in der Zusammensetzung vorliegt, von etwa 1 bis etwa 10 Gew.-% der Zusammensetzung beträgt.

22. Verwendung nach Anspruch 1, wobei die Zusammensetzung in der Form einer Emulsion vorliegt.

23. Verwendung nach Anspruch 2, wobei die Zusammensetzung in der Form einer Emulsion vorliegt.

24. Verwendung nach Anspruch 2, wobei die Zusammensetzung weiter ein Erweichungsmittel umfasst.

25. Verwendung nach Anspruch 2, wobei die Zusammensetzung weiter ein Feuchthaltemittel umfasst.

26. Verwendung nach Anspruch 13, wobei die Zusammensetzung weiter eine oder mehrere Substanzen aus der Gruppe umfasst, bestehend aus: Aufhellungsmitteln, Abdunklungsmitteln, wie etwa Selbstbräunungsmitteln, Antiaknemitteln, Glanzkontrollmitteln, antimikrobiellen Mitteln, entzündungshemmenden Mitteln, antimykotischen Mitteln, Antiparasitenmitteln, externen Analgetika, Sonnenschutzmitteln, Photoprotektoren, Antioxidationsmitteln, keratolytischen Mitteln, Detergentien/Tensiden, Befeuchtungsmitteln, Nährstoffen, Vitaminen, Energieverstärkern, schweißhemmenden Mitteln, adstringierenden Mitteln, Deodorantien, Haarentfernern, haarwachstumsinduzierenden Mitteln, Festigungsmitteln, Antischwielenmitteln, Vitaminen, Bleichmitteln, Entpigmentierungsmitteln, Schaumbildnern, Conditionern, Feuchthaltemitteln, Duftstoffen, Antialterungsmitteln, Antikrebsmitteln, krebsverhindernden Mitteln und Mitteln für Haar-, Nägel- und Hautkonditionierung.

## Revendications

1. Utilisation d'un produit de légumineuse non-dénaturé dans la fabrication d'un médicament pour le traitement ou la prévention des lésions cutanées induites par la radiothérapie, dans laquelle ledit médicament est pour une application sur une zone de la peau du patient qui va être ou a été irradiée avec les rayonnements ionisants conformément à ladite radiothérapie.

2. Utilisation selon la revendication 1, dans laquelle ledit produit de légumineuse est un produit de soja non-dénaturé.

3. Utilisation selon la revendication 1, dans laquelle ledit médicament est pour une application avant l'exposition aux rayonnements.

4. Utilisation selon la revendication 1, dans laquelle ledit médicament est pour une application avant et après l'exposition aux rayonnements.

5. Utilisation selon la revendication 3, dans laquelle ledit médicament est pour une application quotidienne pendant de 1 à 14 jour(s) avant l'exposition aux rayonnements.

6. Utilisation selon la revendication 5, dans laquelle ledit médicament est pour deux applications quotidiennes à au moins 6 heures d'intervalle.

7. Utilisation selon la revendication 4, dans laquelle ledit médicament est pour une application pendant de 1 à 14 jour(s) avant l'exposition aux rayonnements et pendant au moins 30 jours après l'exposition aux rayonnements.

8. Utilisation selon la revendication 1, dans laquelle ledit médicament est pour une application après l'exposition aux rayonnements.

9. Utilisation selon la revendication. 8, dans laquelle ledit médicament est pour une application pendant au moins 30 jours après l'exposition aux rayonnements.

10. Utilisation selon la revendication 8, dans laquelle ledit médicament est pour une application après chaque exposition aux rayonnements.

11. Utilisation selon la revendication 10, dans laquelle ledit médicament est tout d'abord appliqué pendant de 1 à 14 jour(s) avant une première exposition aux rayonnements.

12. Utilisation selon la revendication 11, dans laquelle ledit médicament est ensuite appliqué pendant au moins 30 jours après la fin de l'exposition aux rayonnements.

13. Utilisation selon la revendication 2, dans laquelle ledit produit de soja non-dénaturé est choisi parmi le groupe constitué par : la poudre de haricot de soja, le lait de soja, la poudre de lait de soja, la poudre de haricot de Lima, le lait de haricot de Lima, la poudre de lait de haricot de Lima, la poudre de haricot noir, le lait de haricot noir, la poudre de lait de haricot noir, et les combinaisons de ceux-ci.

14. Utilisation selon la revendication 13, dans laquelle ledit produit de soja non-dénaturé est présent dans la composition en une quantité efficace pour prévenir les lésions cutanées induites par la radiothérapie.

15. Utilisation selon la revendication 14, dans laquelle la quantité de produit de soja non-dénaturé présente dans la composition se situe dans la plage allant d'environ 0,1 à environ 20 % en poids de la composition.

16. Utilisation selon la revendication 15, dans laquelle la quantité de produit de soja non-dénaturé présente dans la composition se situe dans la plage allant d'environ 0,5 à environ 15 % en poids de la composition.

17. Utilisation selon la revendication 16, dans laquelle la quantité de produit de soja non-dénaturé présente dans la composition se situe dans la plage allant d'environ 1 à environ 10 % en poids de la composition.

18. Utilisation selon la revendication 13, dans laquelle ledit produit de soja non-dénaturé est présent dans la composition en une quantité efficace pour traiter les lésions cutanées induites par la radiothérapie.

19. Utilisation selon la revendication 18, dans laquelle la quantité de produit de soja non-dénaturé présente dans la composition se situe dans la plage allant d'environ 0,1 à environ 20 % en poids de la composition.

20. Utilisation selon la revendication 19, dans laquelle la quantité de produit de soja non-dénaturé présente dans la composition se situe dans la plage allant d'environ 0,5 à environ 15 % en poids de la composition.

21. Utilisation selon la revendication 20, dans laquelle la quantité de produit de soja non-dénaturé présente dans la composition se situe dans la plage allant d'environ 1 à environ 10 % en poids de la composition.

22. Utilisation selon la revendication 1, dans laquelle ladite composition est sous la forme d'une émulsion.

23. Utilisation selon la revendication 2, dans laquelle ladite composition est sous la forme d'une émulsion.

24. Utilisation selon la revendication 2, dans laquelle ladite composition comprend en outre un émollient.

25. Utilisation selon la revendication 2, dans laquelle ladite composition comprend en outre un humectant.

26. Utilisation selon la revendication 13, dans laquelle ladite composition comprend en outre un ou plusieurs des éléments du groupe constitué par : les agents éclaircissants, les agents colorants tels que les agents auto-bronzants, les agents anti-acné, les agents de contrôle de la brillance, les agents antimicrobiens, les agents anti-inflammatoires, les agents antimycosiques, les agents antiparasitaires, les analgésiques externes, les écrans solaires, les photo-protecteurs, les antioxydants, les agents kératolytiques, les détergents / agents de surface, les agents hydratants, les agents nutritifs, les vitamines, les agents d'augmentation de l'énergie, les anti-transpirants, les agents astringents, les déodorants, les agents dépilatoires, les stimulateurs de pousse des cheveux, les agents raffermissants, les agents anti-callosités, les agents de blanchissement, les agents de dépigmentation, les agents moussants, les conditionneurs, les humectants, les parfums, les agents antivieillissement, les agents anticancéreux, les agents de prévention du cancer et les agents revitalisants pour les cheveux, les ongles et la peau.
